# EUROPEAN PATENT SPECIFICATION

(11) **EP 3 564 989 B1**
(45) Date of publication and mention of the grant of the patent: **01.02.2023**
(21) Application number: 16923607.2
(22) Date of filing: 08.12.2016
(51) Int. Cl.: H01L 23/15, A61L 9/03, G08C 17/00, A63F 13/28, A61L 9/04, A61L 9/12, H04M 1/21, H04W 88/02

(54) **ELECTROTHERMAL HYBRID MICROSYSTEM FOR CONTROLLED DISPENSING OF FRAGRANCES AND AROMAS**
ELEKTROTHERMISCHES HYBRIDES MIKROSYSTEM ZUR KONTROLLIERTEN ABGABE VON DUFTSTOFFEN UND AROMEN
MICROSYSTÈME HYBRIDE ÉLECTROTHERMIQUE POUR LA DISTRIBUTION RÉGULÉE DE FRAGRANCES ET D'ARÔMES

(30) Priority: 07.12.2016 BR 102016028782
(43) Date of publication of application: 06.11.2019
(73) Proprietor: Instituto De Pesquisas Tecnológicas Do Estado De São Paulo S.A. - IPT, 05508-901 São Paulo (BR); Ananse Química Ltda, 18560-000 Iperó - SP (BR)
(72) Inventor: GALVÃO, Claudia, 18560-000 Ipero (BR); GONGORA RUBIO, Mario Ricardo, 05508-901 São Paulo (BR); PEREIRA CERIZE, Natalia Neto, 05508-901 São Paulo (BR); COBAS GOMEZ, Houari, 05508-901 São Paulo (BR); ARAGAO HOROIWA, Thais, 05508-901 São Paulo (BR); OLIVEIRA, Adriano Marim De, 05508-901 São Paulo (BR); WASNIEVSKI DA SILVA DE LUCA RAMOS, Luciana, 05508-901 São Paulo - SP (BR); RODRIGUES DE CASTRO, Jordana, 05508-901 São Paulo - SP (BR)
(74) Representative: Juncosa Miró, Jaime
(86) International application number: PCT/BR2016/050316
(87) International publication number: WO 2018/102893

(56) References cited:
- WO-A1-2013/001972
- WO-A1-2017/100874
- BR-A- 9 206 449
- CN-A- 103 675 040
- CN-A- 105 920 644
- CN-A- 105 957 326
- US-A1- 2004 204 043
- US-A1- 2010 176 213
- US-A1- 2015 048 178
- US-A1- 2015 374 870

## Description

### FIELD OF APPLICATION

The Invention, belonging to the olfactory experimentation sector to serve as a showcase for fragrances, concerns the micromanufacture of microsystems that integrated multiple functionalities including electrothermal actuators and radio frequency transmitters/receivers, as well as the manufacture of microsystems responsive to wireless radio frequency signals and the manufacture of microsystems that control the release of fragrances and/or aromas activated by precisely controlling variations in temperature, through the activation of thermal resistance controlled by wireless.

### STATE OF THE ART

A microsystem can be defined as a set of interconnected elements that function as a structurally constituted whole, capable of handling at least two media, for example electricity and liquids, by means of sensors, actuators, interconnections and electronic signal and control processes, where the integration is realized by means of devices of significantly reduced dimensions. Currently, techniques are applied using multiple ceramic layers in LTCC *(Low Temperature Cofired Ceramic)* to construct integrated circuits that can be used in MST *(Micro System Technology)* for the construction of electronic and non-electronic microsystems.

Different ceramic substrates have been successfully used in microelectronics in the production of printed circuits, as well as in the production of sensors, actuators and passive components. The electronic ceramics industry, for example, has developed substrate technology which, applied in the microelectronics industry, provides sophisticated applications for the interconnection of electronic systems of up to 40 multilayers, which can be stacked to form a single body. This system is compatible with thick-film technology, thus allowing for the deposition on the ceramic tapes of conductive, resistive and dielectric films in accordance with the application. Multilayered technology in LTCC provides a material with low dielectric loss, stable dielectric constant and 30 functionalization that allows for its use in MCM's *(Multi Chip Module)* and in the wireless communication industry, for example, in the production of Bluetooth modules, where it is necessary that the device is sufficiently compact and versatile to fit into other small devices.

The materials used to obtain green ceramic tapes in LTCC technology are vitroceramic with very uniform grain structures. The raw material is alumina (Al203) with a vitrea matrix whose objective is to reduce the temperature during the viscous sintering of the system, rendering the material compatible with thick-film technology, and these tapes have thicknesses of 50 to 300 um. LTCC technology allows for the generation of mechanical structures (boreholes, beams, bridges, channels, cavities) in intermediate sizes in the range of 100 um to several millimeters, and in a simple manner. The tapes are easily manipulated when still green, being soft, flexible, and easily dissolved and burned. For the micromanufacture of the devices in LTCC the steps below are followed:
- Molding (lithography, micromachining by UV Laser) of the ceramic tapes for the opening of pathways, channels, cavities, etc.;
- Deposition of thick-film resistors/conductors/dielectrics on the surface or buried using precision screen-printing techniques;
- Lamination of the ceramic tapes to obtain a multilayer system;
- Sintering of the laminate;
- Electronic circuit assembly (transmission/receiving system, system for controlling thermal actuators) and realization of the electrical interconnection.

Demonstrably, olfactory marketing is an excellent tool for increasing sales in all segments, for example: perfumes, cosmetics and food, where chocolate and coffee aromas, for example, can increase yields by up to 30% during the release of these aromas in stores, corridors of shopping malls and at promotional events. Even brands unrelated to the food segment have sought to associate a certain fragrance or aroma with their products.

The current state of the art anticipates some patent documents that cover the material in question, such as PI 9206449-3 ("Sampler for Releasing Encapsulated Liquid Material and Process for Obtaining it") which concerns microencapsulated fragrances glued to a page, whose inventive activity is based on the use of a protective treatment between the printing inks on the paper and the layer containing the microcapsules or their associated adhesion layers if present.

The technology, although widely used by the market, presents a series of limitations and disadvantages such as excessive paper costs in the manufacture of catalogs, packaging, products, the industrial manufacturing and disposal process, in addition to the impossibility of combining the fragrances or aromas contained in the catalogues.

Currently, there is a strong tendency to develop olfactory experiences on mobile devices, delivered through a combination of *software* and *hardware,* allowing for the integration of aromas in electronic products.

The most innovative solutions are already offered by the market, such as the Scentee® Smartphone Aroma Diffusor (http://thegadgetflow.com/portfolio/scentee-smartphone-aroma-diffuser, accessed on October 11, 2016) that consists of a device connected to the headset of a *smartphone,* which has the function of releasing odors. This equipment allows the user, when receiving *e-mails* and notifications from social networks, to smell essences and aromas, but a negative factor is that the 'p2' connector of the smartphone is a technology that is already being discontinued, in addition to the fact that it eliminates the possibility of concomitant audition, since it is used by the equipment releasing the fragrances and/or aromas. The patent document WO 2013001972 *(Devide, method and program for message exchange and conversation via network)* reveals the aforementioned aroma-releasing device applied to devices for exchanging messages that emits an aroma in accordance with the messages received via the *web,* but there is also a need for a physical connection with the device that generates the signal.

The "electrothermal hybrid microsystem for the controlled release of fragrances and aromas" differs from this device because it does not require a physical connection with the mobile device, bearing in mind that it communicates through a wireless signal with the device issuing the command for the release of the fragrance. *In addition to this, there is* a *precise control of the temperature achieved and maintenance thereof which ensures the release of fragrances and aromas without alteration of the olfactory characteristics. Without the control, alterations occur in the fragrances and*/*or aromas, with the consequent distortion of the smells.* Document US 2015/0048178 *(Systems, methods and articles to provide olfactory sensations)* contains a description of a device that can capture aromas from the environment and release them in a controlled manner using a configurable system associated with a Peltier heating or cooling effect which releases the retained aroma. This device has, as a negative aspect, the fact that the system that emits the electrical stimulus is not integrated into the odor emitting actuator system, and is presented in two separate modules that are interconnected by a cable. Moreover, there is no precise temperature control, and alterations can occur in the scent of the fragrances and aromas.

Another document, No. BR2015/050257, concerns a device for releasing fragrances and aromas activated by wireless technology based on the phenomenon of volatilization through an increase of temperature in resistances, or by mechanical rupture or, moreover, by ultrasound and a ventilation system that distributes the odor through a fabric located in the middle of the mechanism. This technology includes electronic media that allow for the release of the fragrance, however, due to its constructivity, its assembly and operation are greatly limited, since the thickness of the device of the above document is greater than 5mm, differentiating it from the proposal conceived in the present invention of an "electrothermal hybrid microsystem for controlled release of fragrances and aromas", which is not greater than 2 mm, thus increasing its versatility. There is also no precise control of the temperature, and distortion of the olfactory experience may occur due to alterations in the smells of the fragrances and aromas.

The present invention for an "electrothermal hybrid microsystem for controlled release of fragrances and aromas", consists of a microsystem that simultaneously integrates a thermal control electronic system, a wireless electronic transmitter/receiver system, an electrical power supply system and a compartment for storing the fragrance and/or aroma, all of which micromanufactured systems possess a platform of reduced thickness, introducing novelty and inventiveness, since this allows the final device to be presented in different formats, to be coupled or connected to smartphones, tablets, laptops or other portable electronic devices for personal use due to the versatile association of the microsystem hereby developed.

US2004204043 A1 provides an information processing apparatus with an interactive scent interface that comprises a housing, a processing module installed in the housing for controlling the information processing apparatus, an input module installed in the housing and electrically connected to the processing module for receiving input signals to generate corresponding control signals and for transmitting the corresponding control signals to the processing module. A display device is installed in the housing and electrically connected to the processing module for transforming information transmitted from the processing module into video data and for displaying the video data. A scent control circuit is also installed in the housing and electrically connected to the processing module for generating a scent control signal and a scent releasing module is installed in the housing and electrically connected to the scent control module for releasing scent particles according to the scent control signal. US2015374870 A1 provides a mobile power pack with fragrance feature is disclosed. An outer surface of the housing is formed with an accommodating space, and a power module is installed in the housing. A heating element is disposed in the housing and corresponding to the accommodating space, and the heating element is electrically connected with the power module. A fragrant shim is disposed in the accommodating space, and the fragrant shim is heated by the heating element for producing aromas.

### SUMMARY OF THE INVENTION

The present invention is defined by an electrothermal hybrid microsystem for the controlled dispensing of fragrances and aromas according to the appended claim 1. Further embodiments are defined by the appended dependent claims.

The proposed electrothermal hybrid microsystem includes a thermal electronic system, a wireless transmitter/receiver, an electrical power supply system, and at least one compartment for storing a fragrance and/or aroma. The microsystem comprises several supports made of LTCC that are stacked, aligned, laminated and sintered together via a viscous flow process, forming a LTCC multi-layered platform. The thermal electronic system, the wireless transmitter/receiver, the electrical power supply system and the compartment for storing the fragrance and/or aroma are integrated on the LTCC multi-layered platform. The thermal electronic system comprises a set of conductors, interconnected with a set of resistors, the set of resistors being printed on one of the supports of the LTCC multi-layered platform within the space delimited for the deposit of the fragrance using a screen printing technique. The electrical power supply system comprises mechanical electrical energy generators or light generators or chemical generators. The at least one compartment for storing the fragrance and/or aroma is open, semisealed or sealed wells by membranes, and the fragrance and/or aromas are in free form or associated with other supporting materials such as solvents or waxes or rubbers or silicas.

In an embodiment, the set of conductors are of silver or silver palladium pastes and the resistors are of Ruthenium dioxide (RU02) and/or Bismuth ruthenium oxide (Bi2Ru2 07); the microsystem is configured to be activated by a wireless radio frequency signal that is received by the wireless transmitter/receiver, the wireless radio frequency signal being Bluetooth; and the mechanical electrical energy generators are capacitors, or the light generators are photovoltaic plates, or the chemical generators are batteries or flexible membrane batteries or coin cell type batteries.

In an embodiment, the supports are in the form of plates.

In an embodiment, the LTCC multi-layered platform with the thermal electronic system has a thickness of 1.33 mm and 30.5 mm on its side.

In an embodiment, the microsystem is coupled or connected to a portable electronic device, wherein volatilization of the fragrance and/or aroma occurs by means of a command foreseen in the portable electronic device that triggers the thermal electronic system by wireless means.

In an embodiment, a fairing of the microsystem further includes a plurality of orifices, wherein volatilization of the fragrance and/or aroma is dispersed through the orifices and, from those, to the environment.

### PURPOSE OF INVENTION

An objective of the present invention is to provide an "electrothermal hybrid microsystem for the controlled release of fragrances and aromas" which basically consists of a microsystem which integrates multiple functionalities containing electrothermal actuators and radio frequency transmitters/receivers for the controlled release of fragrances and aromas preserving their olfactory characteristics through precise control of the temperature achieved.

An objective of the present invention is to provide a microsystem which integrates multiple functionalities based on micromanufactured media associated with an electrothermal hybrid system which, through the application of a voltage to a set of conductors associated with resistors, causes the controlled release of fragrances and aromas by the action of the heat generated in these resistors.

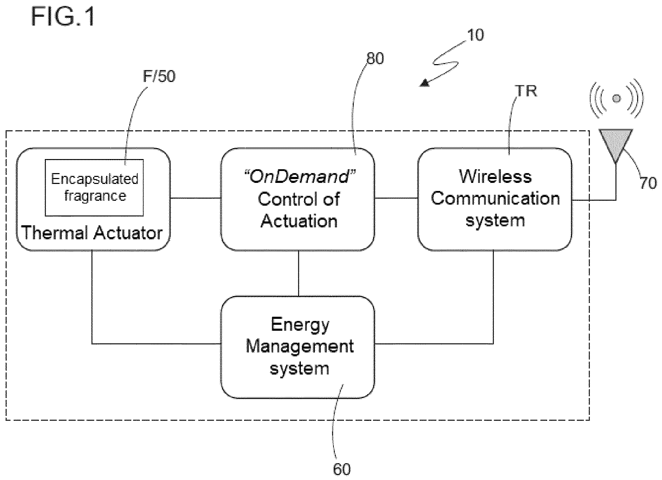

The "electrothermal hybrid microsystem for the controlled release of fragrances and aromas" is also intended for the construction of micromanufactured supports, which can be, but not strictly, built beneath an LTCC platform, integrated with electrothermal systems capable of producing a controlled thermal variation from an electrical stimulus, commanded by an electronic device that communicates through a wireless signal, as shown in Figure 1.

It is also an objective of the present invention to produce associations of fragrances and/or aromas with electrothermal systems built on different micromanufactured platforms that respond to the thermal variation produced by this system, undergoing the phenomenon of controlled volatilization, traversing, or not, membranes with known porosity, and dissipating through the environment so that the odor of the fragrance and/or aroma is perceptible only when the user activates the invention using an electronic device.

It is also an objective of the present invention to construct a system for releasing fragrances and/or aromas on a micromanufactured platform that is responsive to an external command from an electronic device that communicates with an electronic controller built into the platform through a wireless signal.

The "electrothermal hybrid microsystem for the controlled release of fragrances and aromas" is also intended to provide a platform to control the release of fragrances and/or aromas responsive to a wireless signal that is sufficiently compact to be easily coupled to portable electronic devices, i.e. with a thickness of less than 5 millimeters.

It is an objective of this invention that the electronic device endowed with, or connected to, the electrothermal hybrid microsystem in question can act as a device for olfactory experimentation, such as a tool for the online exhibition and commercialization of perfumes and other products that are associated with aromas and odors.

By way of example, a mobile electronic device of the tablet type, endowed with, or coupled to, the electrothermal hybrid microsystem in question, can replace perfume catalogs and equivalents of the type used by thousands of direct sales dealers, very common in mono or multi-level systems, thus avoiding, for example, the printing of millions of paper catalogs with microencapsulated fragrances glued onto them. The catalogs are printed at intervals of 20 to 30 days.

An objective of this invention is that the mobile electronic device can receive or connect to at least one electrothermal hybrid microsystem with at least four wells of the same fragrance so that from each well, following the external stimulus chosen by the user, the controlled release of said desired fragrance and aroma can occur. And it is also an objective that each mobile electronic device includes multiple electrothermal hybrid microsystems with different fragrances, guaranteeing a diverse range of aromas so that the user can access said chosen fragrance, through external stimuli, e.g., a key or icon of the device, or, moreover, through an RF signal and other activation stimuli.

### DESCRIPTION OF THE FIGURES

Figure 1 -Shows a schematic drawing of a block diagram of the constituents of the "electrothermal hybrid microsystem for the controlled release of fragrances and aromas";

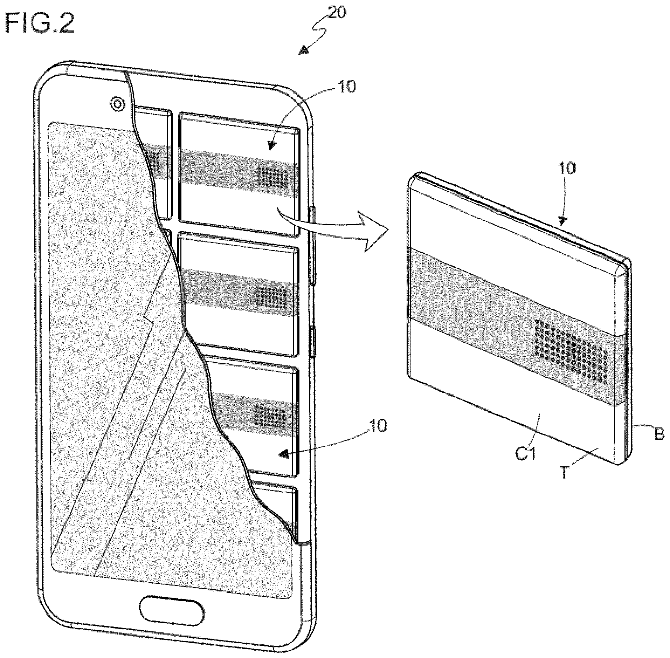

Figure 2 - Shows a symbolic drawing of a mobile electronic device, for example a smartphone, with multiple electrothermal hybrid microsystems for the controlled release of fragrances and aromas housed in their fairings;
Figure 3 shows a microsystem that includes a micro-plastic case in an open position and in perspective, and an LTCC plate with the drawing of the printed conductors and resistors;

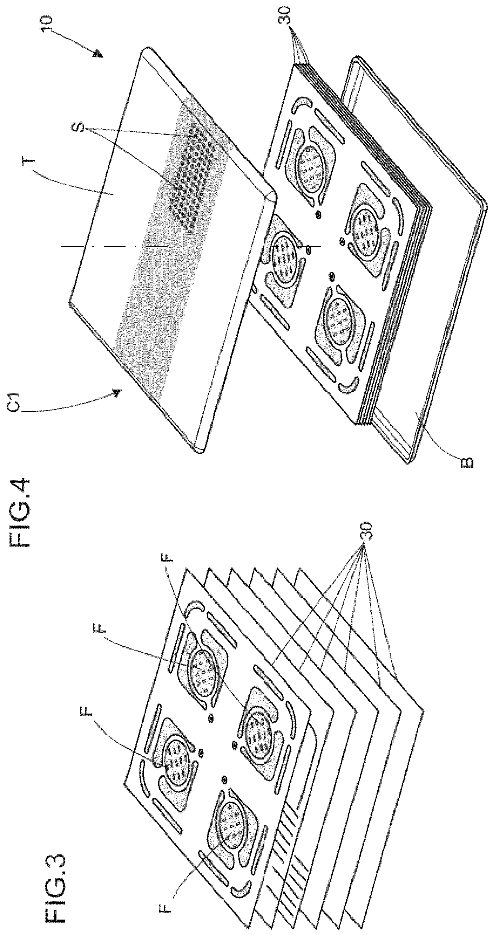

Figure 3 illustrates the micromanufactured supports or platforms that compose the LTCC plate of the microsystem of this invention;
Figure 4 shows, by way of example, the micro-manufactured LTCC plate positioned between the base and the cover of the protective fairing that composes the microsystem;

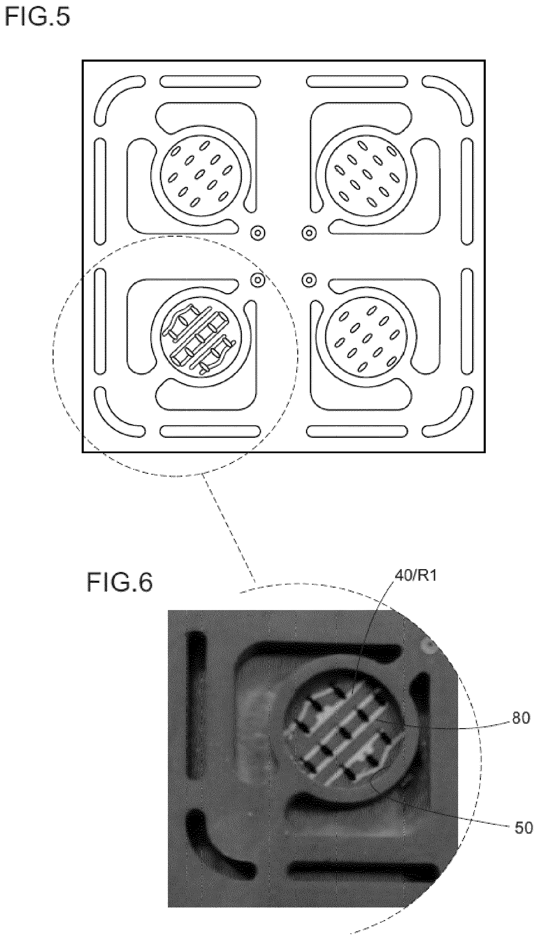

Figure 5 - Schematic drawing and upper view of the LTCC plate with the drawing of the printed conductors and resistors;
Figure 6 - Schematic drawing in upper view of the micro-manufactured wells on the LTCC plate with printed resistors and conductors;

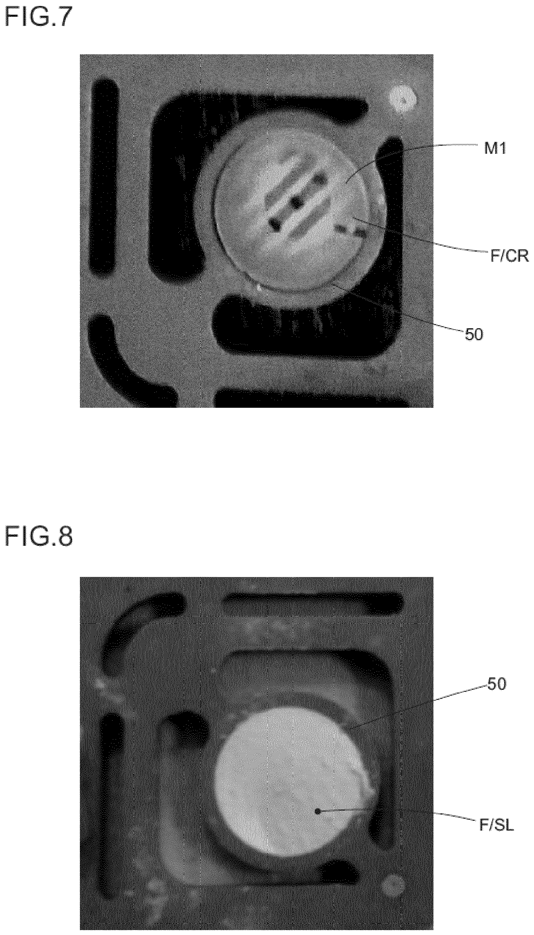

Figure 7 - Schematic drawing in upper view of the LTCC plate filled with a mixture of wax and fragrance;
Figure 8- Schematic drawing in upper view of the LTCC plate filled with a mixture of fragrance and micronized silica;

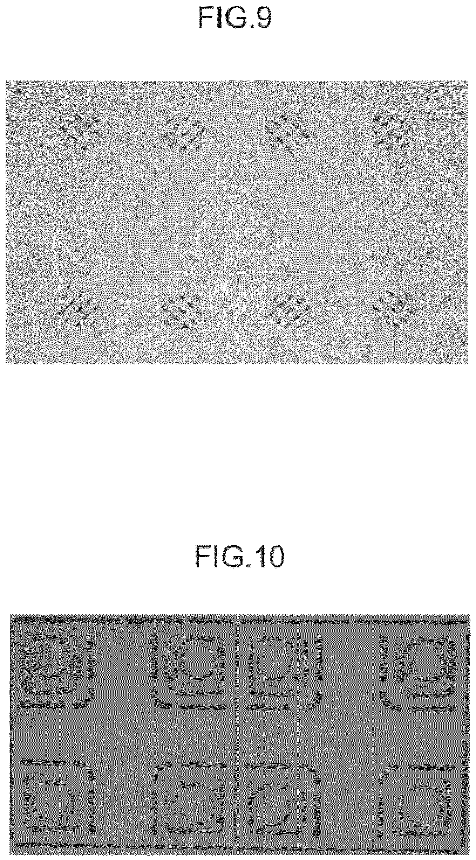

Figure 9- Schematic drawing of the printed resistors on the green ceramic plate (4);
Figure 10- Schematic drawing in upper view of green ceramic plate cut and stacked in stacking orientors;

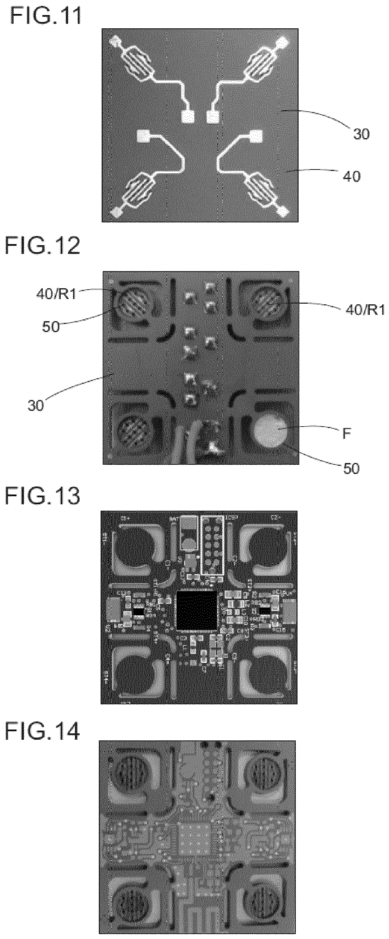

Figure 11- Schematic drawing in upper view of sintered green ceramic plate (LTCC) with printed electronic circuits;
Figure 12- Schematic drawing in upper view of sintered green ceramic plate (LTCC) containing connecting wires;
Figure 13 - Schematic drawing of the electrical circuit device on the LTCC plate;
Figure 14 - Schematic drawing in upper view of the LTCC platform; and

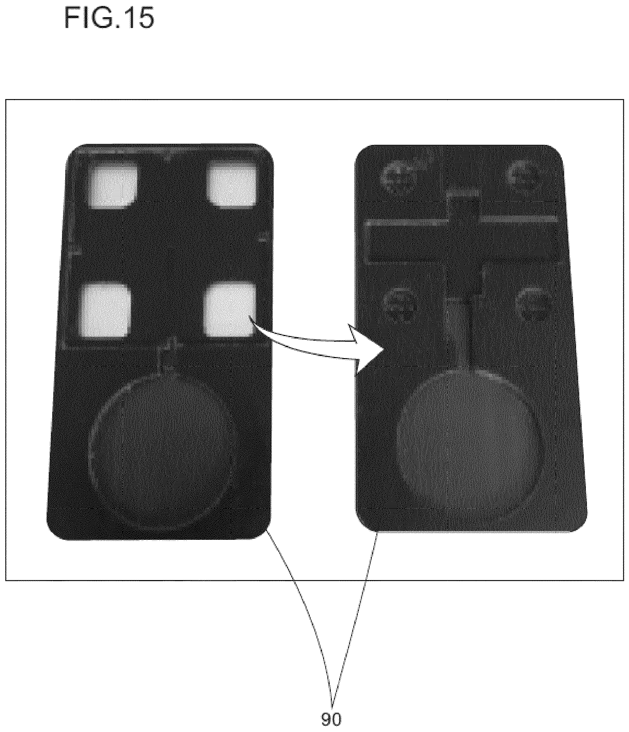

Figure 15- Schematic drawing in upper view of the microsystem containing the fragrance mobilized in its wells inside a plastic case.

### DETAILED DESCRIPTION OF THE INVENTION

Fragrances and aromas are volatile compounds that are perceived by the olfactory organs and trigger a series of metabolic responses in human beings capable of provoking emotional responses associated with this sensory stimulus.

This having been noted, the present invention concerns the construction of a system capable of releasing odors from the volatilization of fragrances and/or aromas promoted by a stimulus produced consciously by the user of the product, in the form of a microsystem (10), coupled or connected to a mobile device (20), of the smartphone, tablet or laptop type, or other portable electronic devices for personal use.

The "electrothermal hybrid microsystem for controlled release of fragrances and aromas" (10) consists of an electrothermal hybrid system built on micro- manufactured supports or platforms (30) (see Figure 3), preferably in the form of plates, which can be glass, fiberglass, thermoresistant plastics or green ceramics (LTCC-Low *Temperature Co-fired Ceramic),* said plates, duly micro-manufactured and joined, composing a unit to be stored in a fairing (C1) with a base (B) and lid (T), where at least one of them has holes (S) for the dispersion of the fragrance or aroma (F), as explained below.

The electrothermal system consists of a set of conductors (40) (Figure 11), which may be, but are not limited to being, silver and silver-palladium pastes, interconnected with a set of resistors (R1), which may be of ruthenium dioxide (Ru02) and/or bismuth ruthenium oxide (Bi2Ru207). These sets of conductors (40) and resistors (R1) can be printed on one of the micro-manufactured supports (30) using the "screen printing" technique within the well (50) delimited for the deposition of the fragrance (F), as shown in figure 12, in accordance with the scheme previously designed in image vectorization software.

The "electrothermal hybrid microsystem for the controlled release of fragrances and aromas" is powered by electrical energy generators (60) (Figure 1), which may be mechanical type generators, such as capacitors, light generators such as solar panels, or chemical generators such as batteries, flexible membrane batteries or, preferably coin cell type batteries.

The fragrance and/or aromas (F) are deposited in the micromanufactured wells (50) of one of the plates (30), as shown in Figures 7, 8 and 12 and may be, but are not limited to being, in free form or associated with other supporting materials such as solvents (ethanol or ethyl acetate), waxes (CR) (carnauba wax, beeswax or candelilla wax), as shown in Figure 7, gums (xanthan gums or gum arabic) or, preferably silica (SL), more preferably micronized silica, as shown in Figure 8.

The wells (50) containing the fragrance and/or aroma (F) may be open, semi- sealed with membranes (M1) with pores of 0.22um to 0.45um which may be cellulose ester or polyvinylidene fluoride - PVDF, or preferably with adhesive tapes of polypropylene and acrylic adhesive, perforated with a pointed tool with a diameter of 0.1 to 0.2um, as shown in Figure 7. The wells (50) may also be sealed with a polytetrafluoroethylene-Teflon film. The "electrothermal hybrid microsystem for controlled release of fragrances and aromas" is activated by a wireless radio frequency signal (70) (Figure 1), preferably Bluetooth, which is received on a transmitter/receiver (TR) integrated into the micro-manufactured platform (10).

The transmitter/receiver (TR), in turn, is integrated into an electronic system of thermal actuators (80) which, on receiving a radio frequency signal (70) from an external electronic device (20), prompt the release of an electrical energy charge through the conductors (40) that is received by the resistors (R1) of the actuator system (80) causing a controlled one-off temperature increase within the wells (50) where the fragrances and aromas (F) are situated.

By increasing the temperature controlled by the autonomic electronic system, the fragrance and/or aroma (F) volatilizes and propagates to the environment through the membranes or perforated adhesive tapes (M1), when present, and the pores built (S) into the fairing (C1), in turn, arranged inside the mobile electronic device (20).

Thus, the odor of the fragrance and/or aroma (F) can be perceived by the user who activated one of the wells (50) of the microsystem (10) through a command foreseen in the electronic device (20), which command, by wireless means, triggers the corresponding electrothermal actuator (80) which, in turn, causes the volatization of the fragrance and aroma (F), dispersing it through the orifices (S) provided in the fairing (C1) of the microsystem (10) and thence to the environment.

### EXAMPLE OF AN EMBODIMENT OF THE INVENTION

### • Manufacture of the microsystem in LTCC

The manufacture of the integrated microsystems (10) began with the printing of the resistive and conductive pastes on green ceramic tapes, as shown in Figure 11, using the precision screen-printing technique, in accordance with previously designed printing screens.

The ceramic leaves printed with the circuits were cut by UV laser in accordance with the desired structure, using previously schematized 30 models, as shown in Figure 10.

Subsequently, the ceramic leaves were stacked, aligned, laminated and sintered in a viscous flow process, applying controlled heat of 6ºC/min until reaching 450°C, which was maintained for 30 minutes and again heated to 850°C at the same rate of 6ºC/min and maintained for 20 minutes, forming a multi-layered LTCC system with a printed electrothermal actuator system, as shown in Figure 9.

The integrated LTCC plate (30) with an electrothermal actuator system (80) has a thickness of 1.33mm and 30.5mm on its side. The four cavities or wells (50), where the fragrances and/or aromas (F) are deposited, have a 5mm internal diameter and a depth of 0.6mm.

Then, the electronic system (figures 13 and 14) is assembled comprising a radio frequency transmitter/receiver (TR) and controller circuits of the electrothermal actuators (80), all integrated into the pre-processed LTCC platform (30), as Illustrated in Figure 13. After the electronic system is printed, the LTCC platform appears as shown in Figure 14, and after preparation of the electronic system, the fragrance (F) is deposited in the micro-manufactured wells (50) in the microsystem (10) mixed with a micronized silica (SL), as shown in Figure 8.

The microsystem (10) containing the fragrance mobilized in its wells (50) is then positioned in a plastic case (90), as shown in Figure 15, remaining stored until its transfer to the fairing (C1), to compose the "electrothermal hybrid microsystem for controlled release of fragrances and aromas" used in a mobile electronic device (20). To evaluate the application in the release of fragrances for validation of the microsystem, the application test was performed with the open microsystem containing the fragrance immobilized with the micronized silica. A known voltage capable of generating current and, consequently, heat in the integrated electrothermal system was applied to the electrical contacts, as shown in Figure 6.

It was observed, through the exhalation of the odor, that the system was able to provoke a release of the fragrance in a controlled manner through the application, or not, of a voltage to the external electrical contact.

## Claims

1. "ELECTROTHERMAL HYBRID MICROSYSTEM FOR THE CONTROLLED DISPENSING OF FRAGRANCES AND AROMAS", including:
a thermal electronic system (80),
a wireless transmitter/receiver (TR),
an electrical power supply system (60), and
at least one compartment (50) for storing a fragrance and/or aroma (F),
wherein:
the microsystem (10) comprises several supports (30) made of Low Temperature Co-Fired Ceramic, LTCC, that are stacked, aligned, laminated and sintered together via a viscous flow process, forming a LTCC multi-layered platform;
the thermal electronic system (80), the wireless transmitter/receiver (TR), the electrical power supply system (60) and the compartment (50) for storing the fragrance and/or aroma (F) are integrated on the LTCC multi-layered platform;
the thermal electronic system (80) comprises a set of conductors (40), interconnected with a set of resistors (R1), the set of resistors (41) being printed on one of the supports (30) of the LTCC multi-layered platform within the space delimited for the deposit of the fragrance using a screen printing technique;
the electrical power supply system (60) comprises mechanical electrical energy generators or light generators or chemical generators;
the at least one compartment (50) for storing the fragrance and/or aroma (F) is open, semisealed or sealed wells by membranes (M1); and
the fragrance and/or aromas (F) are in free form or associated with other supporting materials such as solvents or waxes (CR) or rubbers or silicas (SL).

2. "ELECTROTHERMAL HYBRID MICROSYSTEM FOR THE CONTROLLED DISPENSING OF FRAGRANCES AND AROMAS", in accordance with claim 1, wherein:
the set of conductors (40) are of silver or silver palladium pastes and the resistors (R1) are of Ruthenium dioxide (RU02) and/or Bismuth ruthenium oxide (Bi2Ru2 07);
the microsystem (10) is configured to be activated by a wireless radio frequency signal (70) that is received by the wireless transmitter/receiver (TR), the wireless radio frequency signal (70) being Bluetooth;
the mechanical electrical energy generators (60) are capacitors, or the light generators are photovoltaic plates, or the chemical generators are batteries or flexible membrane batteries or coin cell type batteries.

3. "ELECTROTHERMAL HYBRID MICROSYSTEM FOR THE CONTROLLED DISPENSING OF FRAGRANCES AND AROMAS", according to claim 1, wherein the supports (30) are in the form of plates.

4. "ELECTROTHERMAL HYBRID MICROSYSTEM FOR THE CONTROLLED DISPENSING OF FRAGRANCES AND AROMAS", according to any one of the previous claims, wherein the LTCC multi-layered platform with the thermal electronic system (80) has a thickness of 1.33 mm and 30.5 mm on its side.

5. "ELECTROTHERMAL HYBRID MICROSYSTEM FOR THE CONTROLLED DISPENSING OF FRAGRANCES AND AROMAS", according to any one of the previous claims, wherein the microsystem (10) is coupled or connected to a portable electronic device (20), wherein volatilization of the fragrance and/or aroma (F) occurs by means of a command foreseen in the portable electronic device (20) that triggers the thermal electronic system (80) by wireless means.

6. "ELECTROTHERMAL HYBRID MICROSYSTEM FOR THE CONTROLLED DISPENSING OF FRAGRANCES AND AROMAS", according to claim 5, wherein a fairing (C1) of the microsystem (10) further includes a plurality of orifices (S), wherein volatilization of the fragrance and/or aroma (F) is dispersed through the orifices (S) and, from those, to the environment.

## Patentansprüche

1. "ELEKTROTHERMISCHES HYBRIDES MIKROSYSTEM ZUR KONTROLLIERTEN ABGABE VON DUFTSTOFFEN UND AROMEN", welches Folgendes einschließt:
ein thermoelektronisches System (80),
einen drahtlosen Sender/Empfänger (TR),
ein Stromversorgungssystem (60), und
mindestens ein Fach (50) zur Aufbewahrung eines Duftstoffes und/oder Aromas (F),
wobei:
das Mikrosystem (10) mehrere Träger (30) aus Niedertemperatur-Einbrand-Keramik, NTEK, umfasst, die über ein viskoses Fließverfahren gestapelt, ausgerichtet, laminiert und zusammengesintert werden, wodurch eine mehrlagige NTEK -Plattform gebildet wird;
das thermoelektronische System (80), der drahtlose Sender/Empfänger (TR), das Stromversorgungssystem (60) und das Fach (50) zur Aufbewahrung des Duftstoffs und/oder Aromas (F) auf der mehrlagigen NTEK-Plattform integriert sind;
das thermoelektronische System (80) einen Satz von Leitern (40) umfasst, die mit einem Satz von Widerständen (R1) verbunden sind, wobei der Satz von Widerständen (41) auf einen der Träger (30) der mehrlagigen NTEK-Plattform innerhalb des für die Ablagerung des Duftstoffs begrenzten Raums unter Verwendung einer Siebdrucktechnik aufgedruckt ist;
das Stromversorgungssystem (60) mechanische elektrische Energiegeneratoren oder Lichtgeneratoren oder chemische Generatoren umfasst;
das mindestens eine Fach (50) zur Aufbewahrung des Duftstoffs und/oder Aromas (F) offen, halbabgedichtet oder durch Membranen (M1) abgedichtet ist; und
der Duftstoff und/oder die Aromen (F) in freier Form vorliegen oder mit anderen Trägermaterialien wie Lösungsmittel oder Wachse (CR) oder Kautschuke oder Kieselerden (SL) verbunden sind.

2. "ELEKTROTHERMISCHES HYBRIDES MIKROSYSTEM ZUR KONTROLLIERTEN ABGABE VON DUFTSTOFFEN UND AROMEN" nach Anspruch 1, wobei:
der Satz von Leitern (40) aus Silber- oder Silberpalladiumpasten und die Widerstände (R1) aus Rutheniumdioxid (RU02) und/oder Bismut-Rutheniumoxid (Bi2Ru2 07) bestehen;
das Mikrosystem (10) so ausgebildet ist, dass es durch ein drahtloses Hochfrequenzsignal (70) aktiviert wird, das von dem drahtlosen Sender/Empfänger (TR) empfangen wird, wobei das drahtlose Hochfrequenzsignal (70) Bluetooth ist;
die mechanischen elektrischen Energiegeneratoren (60) Kondensatoren sind, oder die Lichtgeneratoren photovoltaische Platten sind, oder die chemischen Generatoren Batterien oder flexible Membranbatterien oder Batterien vom Typ Knopfzelle sind.

3. "ELEKTROTHERMISCHES HYBRIDES MIKROSYSTEM ZUR KONTROLLIERTEN ABGABE VON DUFTSTOFFEN UND AROMEN" nach Anspruch 1, wobei die Träger (30) in Form von Platten vorliegen.

4. "ELEKTROTHERMISCHES HYBRIDES MIKROSYSTEM ZUR KONTROLLIERTEN ABGABE VON DUFTSTOFFEN UND AROMEN" nach einem der vorhergehenden Ansprüche, wobei die mehrlagige NTEK-Plattform mit dem thermoelektronischen System (80) eine Stärke von 1,33 mm und 30,5 mm an ihrer Seite aufweist.

5. "ELEKTROTHERMISCHES HYBRIDES MIKROSYSTEM ZUR KONTROLLIERTEN ABGABE VON DUFTSTOFFEN UND AROMEN" nach einem der vorhergehenden Ansprüche, wobei das Mikrosystem (10) mit einer tragbaren elektronischen Vorrichtung (20) gekoppelt oder verbunden ist, wobei die Verflüchtigung des Duftstoffs und/oder des Aromas (F) mittels eines in der tragbaren elektronischen Vorrichtung (20) vorgegebenen Befehls erfolgt, der das thermoelektronische System (80) auf drahtlosem Wege auslöst.

6. "ELEKTROTHERMISCHES HYBRIDES MIKROSYSTEM ZUR KONTROLLIERTEN ABGABE VON DUFTSTOFFEN UND AROMEN" nach Anspruch 5, wobei eine Verkleidung (C1) des Mikrosystems (10) ferner eine Vielzahl von Öffnungen (S) einschließt, wobei die Verflüchtigung des Duftstoffs und/oder des Aromas (F) durch die Öffnungen (S) und von diesen an die Umgebung abgegeben wird.

## Revendications

1. « MICROSYSTÈME HYBRIDE ÉLECTROTHERMIQUE POUR LA DISTRIBUTION CONTRÔLÉE DE PARFUMS ET DE SENTEURS », comprenant :
un système électronique thermique (80),
un transmetteur/récepteur sans fils (TR),
un système d'alimentation en courant électrique (60), et
au moins un compartiment (50) pour stocker un parfum et/ou une senteur (F), dans lequel :
le microsystème (10) comporte plusieurs supports (30) faits en Céramique Cocuite à Basse Température, LTCC, qui sont empilés, alignés, stratifiés et frittés ensembles par le biais d'un processus d'écoulement visqueux, formant une plateforme à couches multiples de LTCC ;
le système électronique thermique (80), le transmetteur/récepteur sans fils (TR), le système d'alimentation en courant électrique (60) et le compartiment (50) pour stocker le parfum et/ou senteur (F) sont intégrés dans la plateforme à couches multiples de LTCC ;
le système électronique thermique (80) comporte un ensemble de conducteurs (40) interconnectés avec un ensemble résistances (R1), l'ensemble de résistances (41) étant imprimé sur l'un des supports (30) de la plateforme à couches multiples de LTCC à l'intérieur de l'espace délimité pour le dépôt du parfum en utilisant une technique de sérigraphie ;
le système d'alimentation en courant électrique (60) comporte des générateurs d'énergie électrique mécaniques ou des générateurs de lumière ou des générateurs chimiques ;
l'au moins un compartiment (50) pour stocker le parfum et/ou la senteur (F) est ouvert, des creux semi-scellés ou scellés par des membranes (M1) ; et
le parfum et/ou les senteurs (F) ont une forme libre ou associée à d'autres matériaux de support tels que des solvants ou des cires (CR) ou des caoutchoucs ou des silices (SL).

2. « MICROSYSTÈME HYBRIDE ÉLECTROTHERMIQUE POUR LA DISTRIBUTION CONTRÔLÉE DE PARFUMS ET DE SENTEURS», conformément à la revendication 1, dans lequel :
l'ensemble des conducteurs (40) est en pâtes d'argent ou de palladium-argent et les résistances (R1) sont en dioxyde de ruthénium (RU02) et/ou en oxyde de ruthénium et de bismuth (Bi2Ru2 07) ;
le microsystème (10) est configuré pour être activé par un signal de radiofréquence sans fils (70) qui est reçu par le transmetteur/récepteur sans fils (TR), le signal de radiofréquence sans fils (70) étant Bluetooth ;
les générateurs d'énergie électrique mécaniques (60) sont des condensateurs ou les générateurs de lumière sont des plaques photovoltaïques ou les générateurs chimiques sont des batteries ou des batteries à membrane souple ou des piles bouton.

3. « MICROSYSTÈME HYBRIDE ÉLECTROTHERMIQUE POUR LA DISTRIBUTION CONTRÔLÉE DE PARFUMS ET DE SENTEURS », conformément à la revendication 1, dans lequel les supports (30) ont la forme de plaques.

4. « MICROSYSTÈME HYBRIDE ÉLECTROTHERMIQUE POUR LA DISTRIBUTION CONTRÔLÉE DE PARFUMS ET DE SENTEURS », conformément à une quelconque des revendications précédentes, dans lequel la plateforme à couches multiples de LTCC ayant le système électronique thermique (80) à une épaisseur de 1,33 mm et 30,5 mm sur ses côtés.

5. « MICROSYSTÈME HYBRIDE ÉLECTROTHERMIQUE POUR LA DISTRIBUTION CONTRÔLÉE DE PARFUMS ET DE SENTEURS », conformément à une quelconque des revendications précédentes, dans lequel le microsystème (10) est couplé ou connecté à un dispositif électronique portable (20), dans lequel la volatilisation du parfum et/ou senteur (F) a lieu au moyen d'une commande prévue sur le dispositif électronique portable (20) qui déclenche le système électronique thermique (80) par un moyen sans fils.

6. « MICROSYSTÈME HYBRIDE ÉLECTROTHERMIQUE POUR LA DISTRIBUTION CONTRÔLÉE DE PARFUMS ET DE SENTEURS », conformément à la revendication 5, dans lequel un carénage (C1) du microsystème (10) comprend en outre une pluralité de trous (S), où la volatilisation du parfum et/ou senteur (F) es dispersée à travers les trous (S) et, à travers eux, à l'environnement.
